# EUROPEAN PATENT SPECIFICATION

(11) **EP 2 551 268 B1**
(45) Date of publication and mention of the grant of the patent: **18.01.2017**
(21) Application number: 12175485.7
(22) Date of filing: 09.07.2012
(51) Int. Cl.: C07D 401/04

(54) **Process for the preparation of moxifloxacin hydrochloride and intermediates thereof**
Verfahren zur Herstellung von Moxifloxacinhydrochlorid und Zwischenprodukten davon
Procédé de préparation d'hydrochlorure de moxifloxacine et intermédiaires correspondants

(30) Priority: 29.07.2011 IT TO20110705
(43) Date of publication of application: 30.01.2013
(73) Proprietor: F.I.S. Fabbrica Italiana Sintetici S.p.A., 36075 Alte di Montecchio Maggiore (VI) (IT)
(72) Inventor: Gottardo, Gianni, 36020 Zovencedo, Vicenza (IT); Padovan, Pierluigi, 36075 Montecchio Maggiore, Vicenza (IT); Osti, Sergio, 36100 Vicenza (IT)
(74) Representative: Leganza, Alessandro

(56) References cited:
- EP-A1- 1 992 626
- LALITHA DEVI M ET AL: "A Validated, Specific Stability-Indicating RP-LC Method for Moxifloxacin and Its Related Substances", CHROMATOGRAPHIA ; AN INTERNATIONAL JOURNAL FOR RAPID COMMUNICATION IN CHROMATOGRAPHY, ELECTROPHORESIS AND ASSOCIATED TECHNIQUES, VIEWEG VERLAG, WI, vol. 69, no. 9-10, 21 March 2009 (2009-03-21), pages 993-999, XP019666785, ISSN: 1612-1112
- RAVINDRA KUMAR Y ET AL: "STRUCTURAL IDENTIFICATION AND CHARACTERIZATION OF IMPURITIES IN MOXIFLOXACIN", JOURNAL OF PHARMACEUTICAL AND BIOMEDICAL ANALYSIS, NEW YORK, NY, US, vol. 34, 1 January 2003 (2003-01-01), pages 1125-1129, XP001167148, ISSN: 0731-7085
- FISCHER & C ROBIN GANELLIN J ED - JANOS FISCHER AND C ROBIN GANELLIN: "The chemical evolution of moxifloxacin [extract]", 13 February 2007 (2007-02-13), ANALOGUE-BASED DRUG DISCOVERY, WEINHEIM : WILEY-VCH, PAGE(S) 337, XP009164077, * BAY11-6371; page 337; compound 73 *

## Description

### Technical field of the invention

A process for the preparation of the pharmaceutical active ingredient called Moxifloxacin hydrochloride and synthetic intermediates thereof forms an object of the present invention.

### State of the art

Moxifloxacin is a broad-spectrum fluoroquinolonic antibacterial agent, used for the treatment of respiratory infections (pneumonia, chronic sinusitis, chronic bronchitis) sold in form of hydrochloride by Bayer AG under the name of Avelox® and Avalox®. It is also sold by Alcon Inc. in a low dosage form for ophthalmic use under the name of Vigamox®.

Moxifloxacin, of formula (II) and having the chemical name of 3-quinolinecarboxylic, 1-cyclopropyl-6-fluoro-1,4-dihydro-8-methoxy-7-[(4aS,7aS)-octahydro-6H-pyrrole[3,4-b]pyridine-6-yl]-4-oxo- acid, is characterized by a fluoroquinolonic skeleton, which is common to that of other two antibiotics of the same category (Gatifloxacin and Balofloxacin) and by a side chain constituted by (4aS,7aS)-octahydro-1H-pyrrole[3,4-b]pyridine.

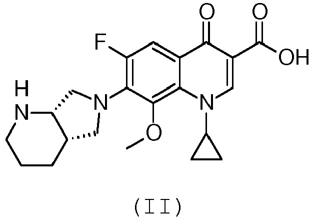

The fluoroquinolonic intermediate of formula (V), with the 8-methoxy group is a commercial product.

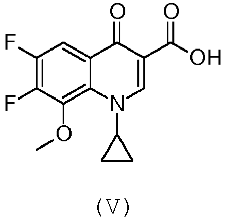

(4aS, 7aS)-Octahydro-1H-pyrrole[3,4-b]pyridine, also referred to as (S,S)-2.8-Diazabycyclo[4.3.0]nonane and having CAS RN [151213-40-0] and formula (VI), 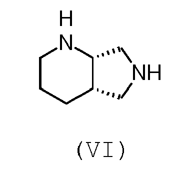 constitutes the side chain of Moxifloxacin and it is the key intermediate of the synthesis in that it has two chiral centers, both having an S configuration, is optically active and levogyre. The preparation of such key intermediate is also described in the two different patent applications MI2009A001353 and WO2010/100215 both owned by F.I.S. Fabbrica Italiana Sintetici S.p.A. which respectively provide for a process for optimising the optical resolution and a regio and stereoselective synthesis process of the bioenzymatic type.

A first synthesis process of Moxifloxacin hydrochloride of the prior art described in EP 550903 comprises the coupling reaction between the 1-Cyclopropyl-6,7-difluoro-1,4-dihydro-8-methoxy-4-oxo-3-quinolinic acid of formula (V): 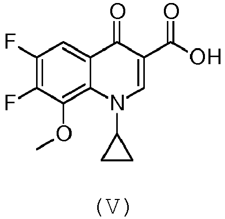 with the (4aS, 7aS)-Octahydro-1H-pyrrole[3,4-b]pyridine intermediate of formula (VI) in presence of a base:

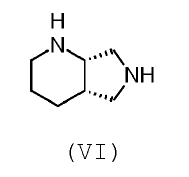

Due to the low regioselectivity of the reaction, the obtained product however contains the 6-isomer impurity of formula (VIII): 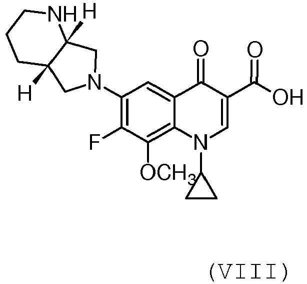 as greater impurity, which - being a position isomer - is difficult to separate from the product, unless to the detriment of consistent yield drops in the product.

The required chromatographic purification on a silica gel column leads to obtaining the same at low yields.

WO 2008/138759 describes a process for the preparation of Moxifloxacin hydrochloride monohydrate where the coupling reaction between 1-Cyclopropyl-6,7-difluoro-1,4-dihydro-8-methoxy-4-oxo-3-quinolinic acid of formula (V) with (S,S)-2.8-diazabycyclo[4.3.0]nonane of formula (VI) is carried out in absence of a base and Moxifloxacin is isolated as L-Tartrate or Fumarate or p-Ditoluyltartrate with the aim of purifying it from the greater impurity, i.e. the 6-isomer of formula (VIII), then it is converted into Moxifloxacin hydrochloride.

A third synthesis process of Moxifloxacin of the prior art described in WO 2005/012285 comprises the reaction between the ethyl ester of 1-Cyclopropyl-6,7-difluoro-1,4-dihydro-8-methoxy-4-oxo-3-quinolinic acid of formula (IX): 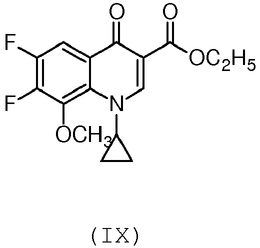 with boric acid and acetic anhydride to form an intermediate borate complex with 95% yield which is reacted with (4aS, 7aS)-Octahydro-1H-pyrrole[3,4-b]pyridine of formula (VI) with 72% yield, then the ester and the complex are hydrolysed, then salification is carried out to give Moxifloxacin hydrochloride with 91% yield, for an overall 62% molar yield.
In WO 2008/059223 a process similar to the previous one is employed where the complex is however generated using boric acid and propionic anhydride instead of acetic anhydride.

A fourth process indicated in WO 2006/134491 provides for the reaction of 1-Cyclopropyl-6,7-difluoro-1,4-dihydro-8-methoxy-4-oxo-3-quinolinic acid of formula (V) with boron trifluoride etherate to give a difluoroborate intermediate which is reacted with the (S,S)-2.8-Diazabycyclo[4.3.0]nonane of formula (VI) in presence of a base with a 92% yield obtaining a difluoroborate complex of Moxifloxacin which is hydrolized and salified to give Moxifloxacin hydrochloride with an overall yield ranging from 42 to 55%.

Furthermore, the synthesis of Moxifloxacin was conducted, as described in EP 1832587, through a process similar to the previous one, carried out one-pot and including silanization before complexation through boron trifluoride.

The processes described above lead to unsatisfactory yields and provides for the use of toxic reagents such as boron trifluoride.

An alternative process which overcomes these problems and especially that of the 6-isomer impurity is the one described in the USA patent application 13/051,081 and Italian patent application MI2010A000450 still owned by F.I.S. Fabbrica Italiana Sintetici S.p.A. where magnesium salt is used for performing the coupling between the 1-Cyclopropyl-6,7-difluoro-1,4-dihydro-8-methoxy-4-oxo-3-quinolinic acid of formula (V) with the intermediate (4aS, 7aS)-Octahydro-1H-pyrrole[3,4-b]pyridine of formula (VI) thus avoiding the formation of the 6-isomer impurity of formula (VIII).

The above-described prior art processes for the preparation of Moxifloxacin in their entirety require the use of coupling between the two syntons described previously. Such coupling also requires the inherent formation of another typical well known impurity of Moxifloxacin referred to as 3-quinolinecarboxylic, 1-cyclopropyl-6-fluoro-1,4-dihydro-8-methoxy-7-[(4aS,7aS)-1-methyloctahydro-6H-pyrrole[3,4-b]pyridine-6-yl]-4-oxo-acid, and having formula (VII): 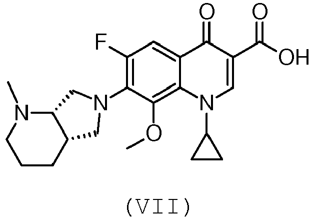 This impurity having the number CAS 721970-37-2 was identified and published for the first time by Dr. Reddy's Laboratories Ltd., Bulk Actives Unit-III, Hyderabad, AP (India) in Journal of Pharmaceutical and Biomedical Analysis (2004), 34(5), 1125-1129 where it is named Impurity-1 and it was observed to be present in amounts above 0.1% in industrially produced Moxifloxacin.
In the posthumous publication of the United States Pharmacopeia-India Private Limited (USP - India) on Chromatographia (2009), 69(9-10), this impurity referred to as Imp-1 was rightfully defined as a process impurity and the samples used by USP - India to carry out the study were provided by Dr. Reddy's Laboratories Ltd. The developed HPLC method allows detecting such impurity in Moxifloxacin with a 0.016% detectability limit.
Both the aforementioned literature published by an important pharmaceutical company and an important authority involved in regulation aspects, as well as the extensive experimentation carried out in our laboratories, confirm that the impurity of formula (VII) is a typical process impurity of Moxifloxacin whose formation is inherently due to the action of hydrofluoric acid, released during the coupling of the two syntons, on the methoxy group in position 8 releasing the methyl carbocation which alkylates a second Moxifloxacin molecule. Furthermore, such impurity is particularly difficult to remove from the product using the conventional re-crystallization methods.

### Summary of the invention

The problem addressed by the present invention is that of providing a process for the preparation of Moxifloxacin hydrochloride capable of allowing limiting the content of 3-quinolinecarboxylic, 1-cyclopropyl-6-fluoro-1,4-dihydro-8-methoxy-7-[(4aS,7aS)-1-methyloctahydro-6H-pyrrole[3,4-b]pyridine-6-yl]-4-oxo-acid of formula (VII) impurity in the product.

Such problem is overcome through a process for the preparation of Moxifloxacin hydrochloride as outlined in the attached claims, whose definitions are an integral part of the present description.

Further characteristics and advantages of the process according to the invention will be apparent from the description outlined below of preferred embodiments, provided by way of non-limiting example.

### Brief description of the figures (or drawings):

By way of example:

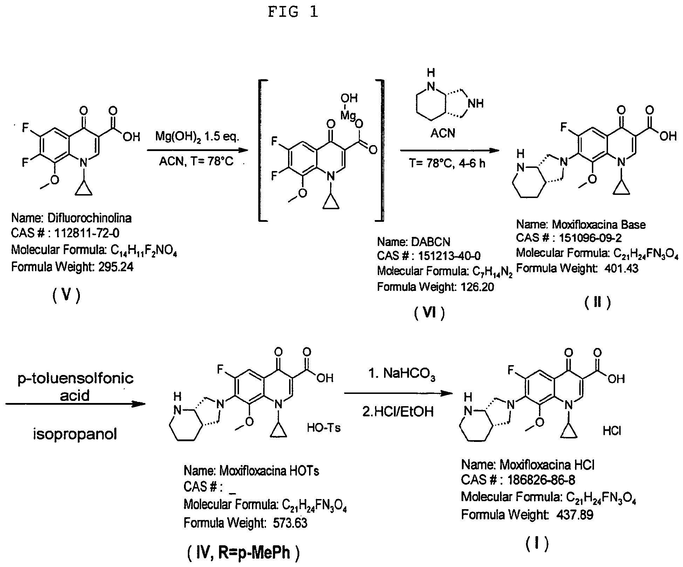

Figure 1 shows the synthesis scheme of Moxifloxacin Hydrochloride according to the preferred aspects of the present invention;

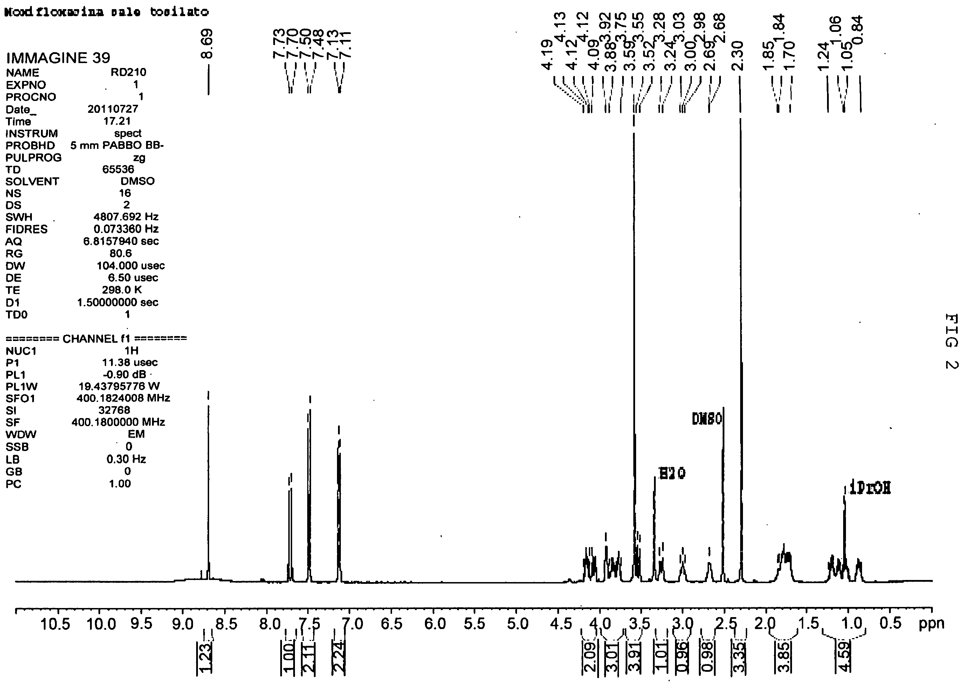

Figure 2 shows the 1H-NMR spectrum of Moxifloxacin Tosylate according to the preferred aspects of the present invention;

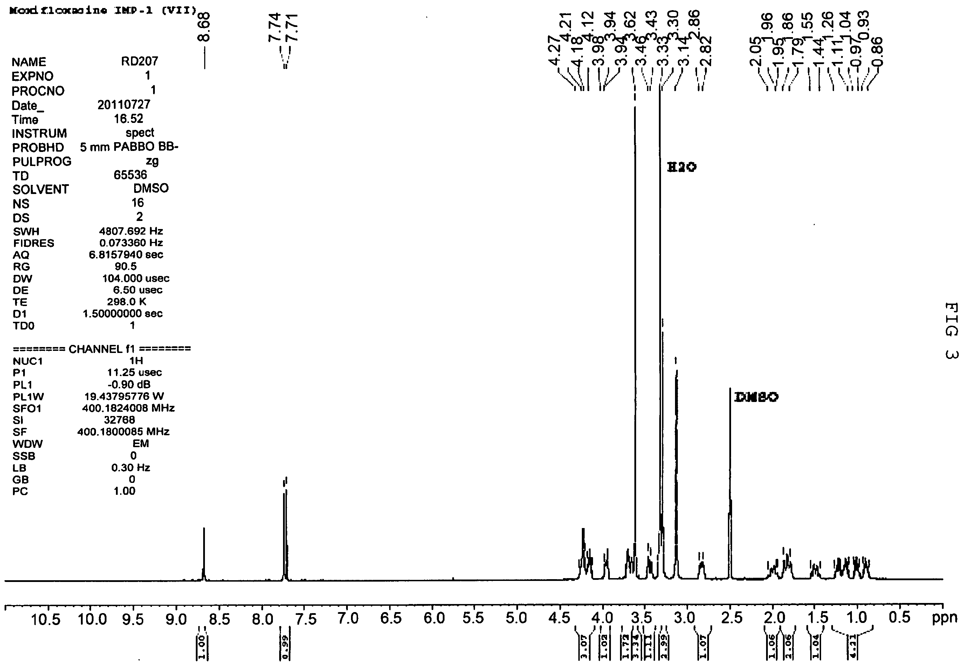

Figure 3 shows the 1H-NMR spectrum of the impurity of Moxifloxacin of formula (VII);
Figure 4 shows the X-Ray Powder Diffraction (XPRD) difractogram of Moxifloxacin Tosylate. Below there are same parameters for the performed analysis.
Figure 5 shows the FT-IR spectrum of Moxifloxacin Tosylate.
Figure 6 shows the Differential Scanning Calorimetry (DSC) curve of Moxifloxacin Tosylate.
Figure 7 shows a picture acquired with the optical microscope of the Moxifloxacin Tosylate crystal powder.

### Detailed description of the invention

The present invention regards a process for the preparation of Moxifloxacin hydrochloride of formula (I) : 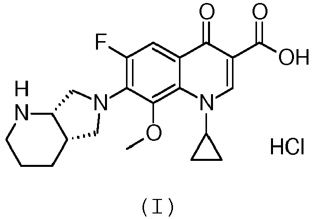 comprising the following steps:
(a) salification of base Moxifloxacin of formula (II) : 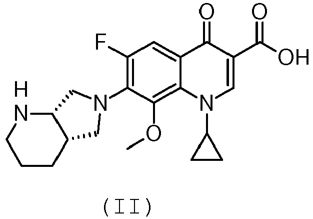 with a sulfonic acid of formula (III):

   R-SO3H (III)

   wherein R is chosen among C1-C4 alkyl, phenyl and tolyl, to give a compound of formula (IV): 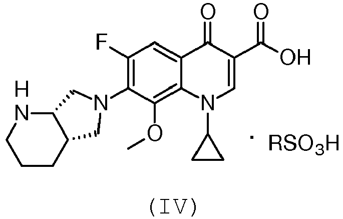
(b) optional purification of the compound of formula (IV),
(c) conversion of the compound of formula (IV) into Moxifloxacin hydrochloride of formula (I).

It was surprisingly discovered that preparing the salts of Moxifloxacin with sulfonic acids alone allows considerably reducing the formula (VII) impurity content in Moxifloxacin and salts thereof.

Actually, as described hereinafter the preparation of other Moxifloxacin salts does not allow purifying the product from the impurity of formula (VII) equivalent to the compound salts of formula (IV) of the present invention, i.e. using the Moxifloxacin salts with sulfonic acids. The preparation of some Moxifloxacin salts different from the sulfonic acids revealed that the amount of such impurity is even increased thus confirming the insolubility of such impurity with respect to Moxifloxacin itself.

The quantification in percentage area of the formula (VII) impurity content in Moxifloxacin and salts thereof may be carried out through the HPLC method indicated in Example 5.

The residue R of the sulfonic acid used according to the process here described is a C1-C12 linear or branched alkyl or an aryl. In particular R alkyl may be a methyl, ethyl, propyl, butyl, pentyl, hexyl, heptyl, octyl, nonyl, decyl, undecyl, docecyl and it may be linear or branched. R may thus also be isopropyl, n-propyl, n-butyl, isobutyl, sec-butyl, n-pentyl, isopentyl, etc. In particular R aryl may be phenyl, tolyl (p-methylphenyl), xylyl, benzyl, naphthyl, indolyl, nosyl, etc.

Preferably R is selected from among C1-C4 alkyl, Phenyl and Tolyl. Even more preferably R is Methyl or Ethyl or Tolyl. Thus, Moxifloxacin mesylate or Moxifloxacin Tosylate are preferred compounds, being the last one the most preferred.

With the aim of carrying out a screening of solvents in which various Moxifloxacin salts are to be prepared, with the final aim of evaluating the capacity thereof to purify Moxifloxacin, the acids indicated in the first column of the table below were used. Preparations were carried out in different alcoholic solvents, as indicated in the first line of table I.

**Table I:**

| ACID | Methanol | Ethanol | Isopropanol | Benzylalcohol |
|---|---|---|---|---|
| Acetic acid | - | - | - | - |
| Formic Acid | - | - | - | - |
| Hydrochloric acid | x | X | x | - |
| Phosphoric Acid | x | x | x | - |
| Oxalic Acid | - | - | x | - |
| Succinic Acid | - | - | - | - |
| Bromidric Acid | x | x | x | x |
| Anhydrous Citric Acid | X | - | - | - |
| Tartaric Acid | - | - | - | - |
| P-toluenesulfonic Acid | X | x | x | - |
| Methanesulfonic Acid | - | x | x | - |

The "-" symbol means that there was no precipitation of the Moxifloxacin salt from the tested solvent, while "X" means that the relative salt was isolated. The experiment was conducted using 5 solvent volumes, 1.05 molar equivalents of acid and carrying out the isolation of the salt by filtration at ambient temperature.

The process of the present invention allows reducing the amount of impurity of formula (VII) in the Moxifloxacin hydrochloride from up to 5% to values comprised in the range from 0.05% to 0.15%. In particular for formula (VII) impurity content comprised between 1.2% and 5% it is required to carry out the optional step (b) with one or more purifications of the compound of formula (IV) while for contents lower than 1.2% the optional step (b) is not typically carried out. The initial Moxifloxacin, obtained through the procedure indicated in example 1, normally has a formula (VII) impurity content comprised between 0.8%-1.2% and thus typically the step (b) does not require to be carried out.

With the process of the present invention Moxifloxacin hydrochloride can thus be obtained comprising between 0.05% and 0.15% of impurity of formula (VII) and preferably between 0.06% and 0.14%, even more preferably comprising between 0.06% and 0.09%, being even more preferable about 0.08% of such impurity.
Step (a) can be carried out starting from a Moxifloxacin base isolated or present in solution, for example in a solution coming from the synthesis thereof by coupling two syntons of formula (V) and (VI). Such step (a) is carried out in an organic solvent, preferably in a C1-C4 alcohol such as Methanol, Ethanol, n-Propanol, Isopropanol, n-Butanol, isobutanol, sec-butanol, tert-butanol; preferably it is carried out in isopropanol. Sulfonic acid may be added in solid form or in a solution of an organic solvent, preferably in the same solvent in which the Moxifloxacin base is solubilised.
Step (b) is optional and provides for the purification of the compound of formula (IV) through one or more re-crystallizations or pulping thereof in an organic solvent, with the possible addition of water. Preferably it is carried out in an isopropanol and water mixture. Preferably it is constituted by one or two pulpings. This optional step allows reducing the amount of impurity of formula (VII) and it can be conveniently used when the initial Moxifloxacin has a formula (VII) impurity content greater than 1.2% (HPLC A%).

The process of the present invention according to step (c) provides for the direct conversion of the Moxifloxacin salts of formula (IV) in Moxifloxacin hydrochloride or such conversion can be carried out with the following two steps:
(c1) conversion of the compound of formula (IV) in Moxifloxacin base of formula (II),
(c2) salification of Moxifloxacin base of formula (II) with hydrochloric acid to give Moxifloxacin hydrochloride of formula (I).

The process for the direct conversion of Moxifloxacin salts of formula (IV) in Moxifloxacin hydrochloride can be carried out in presence of concentrated hydrochloric acid, ethanol and water. The conversion with two steps can be carried out by firstly releasing Moxifloxacin from the salt thereof by extracting in an organic solvent in presence of a base in the aqueous phase. The organic phase may be constituted by a dichloromethane and isopropanol mixture, while the aqueous phase may contain sodium bicarbonate. Subsequently the Moxifloxacin base phase is converted into Moxifloxacin hydrochloride through treatment using concentrated hydrochloric acid in Ethanol.

A further unexpected advantage of the process of the present invention lies in the fact that preparing the salts of formula (IV), besides reducing the formula (VII) impurity content, also allows increasing both the chemical purity and the optical purity of Moxifloxacin. The comparative Table II below summarises the capacity of various Moxifloxacin salts to remove or increase the formula (VII) impurity content in Moxifloxacin and increase the chemical purity thereof. In the first line data are indicated regarding the initial Moxifloxacin that was used.

**Table II:**

| Acid used in step (a) | Moxifloxacin salt obtained | Impurity (VII) HPLC A% | Moxi Purity HPLC A % | A%(VII) salt / A%(VII) Moxi. |
|---|---|---|---|---|
| - | Initial Moxifloxacin | 1.77% | 72.77% | - |
| Hydrochloric acid | Hydrochloride | 1.54% | 93.45% | 87% |
| Phosphoric Acid | Phosphate | 2.31% | 76.34% | 131% |
| Bromidric Acid | Bromohydrate | 2.22% | 86.75% | 125% |
| Oxalic Acid | Oxalate | 1.88% | 87.38% | 106% |
| - | Initial Moxifloxacin | 1.84% | 85.64% | - |
| Naphthalene disulfonic acid | Naphthalenedisulphate (1:1) | 1.85% | 95.06% | 100% |
| Naphthalene disulfonic acid | Naphthalenedisulphate (2:1) | 1.32% | 96.12% | 71% |
| Methanesulfonic acid | Methanesulfonate | 0.78% | 87.26% | 42% |
| Ethanesulfonic Acid | Ethanesulfonate | 0.70% | 98.52% | 38% |
| - | Initial Moxifloxacin | 1.09% | 90.27% | - |
| p-toluenesulfonic acid | Tosylate | 0.54% | 98.64% | 50% |
| - | Initial Moxifloxacin | 1.38% | 89.55% | - |
| p-toluenesulfonic acid | Tosylate | 0.73% | 98.45% | 53% |
| - | Initial Moxifloxacin | 1.79% | 91.01% | - |
| p-toluenesulfonic acid | Tosylate | 0.82% | 98.55% | 46% |

The initial Moxifloxacin having been prepared according to the disclosures of the patent application USA 13/051,081 owned by F.I.S. Fabbrica Italiana Sintetici S.p.A., is free of isomer-6 impurity of formula (VIII) and thus it is not possible to evaluate the possible very probable advantage deriving from the process of the present invention for removing such other typical process impurity.

The preparations of the Moxifloxacin salts indicated in Table II were carried out obtaining the Moxifloxacin base in Isopropanol and adding the corresponding acid at the suitable amounts.

Observing Table II it is evincible that the salts of formula (IV) according to the present description, i.e. the Moxifloxacin salts with sulfonic acids, allow obtaining Moxifloxacin with a low formula (VII) impurity content, typically comprised between 0.5% and 0.9%, well below the one that is obtained for example with the Moxifloxacin salt with oxalic acid, or with hydrobromic acid where the impurity even increases (see the last column with ratio between the amount of the impurity (VII) in the prepared salt and initial one in the Initial Moxifloxacin). The preparation of the Tosylate salt allows reducing the amount of such impurity by about 50%. Also the Moxifloxacin salt of stoichiometry 1 mole of naphtalenedisulfonic acid for two moles of Moxifloxacin called Moxifloxacin Naphtalendisulphonate (2:1), is part of the present invention. Preferably the two sulfonic groups are in position 1 and 5 on the naphthalene ring.

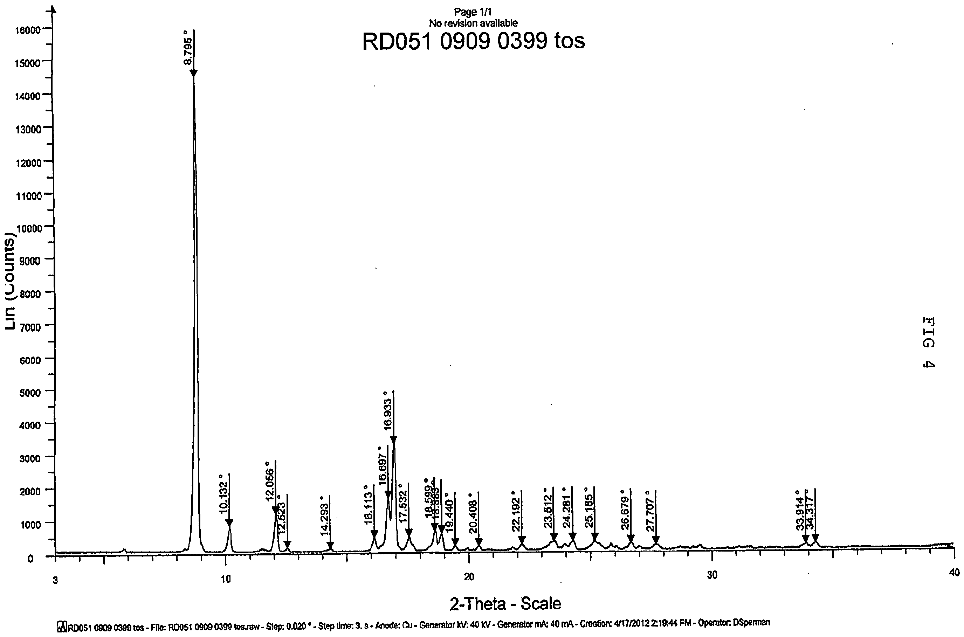

The preparation of Moxifloxacin Tosylate according to the process of the present description is a very preferred embodiment. Moxifloxacin Tosylate prepared according to this method is a crystalline solid which exhibits a characteristic X-Ray Powder Diffraction (XRPD) pattern with characteristic peaks expressed in 2-Theta value: 8.8, 10.2, 12.1, 16.7, 17.0 (+/-) 0.1 (see Fig. 4). We named this crystalline form as Form I.

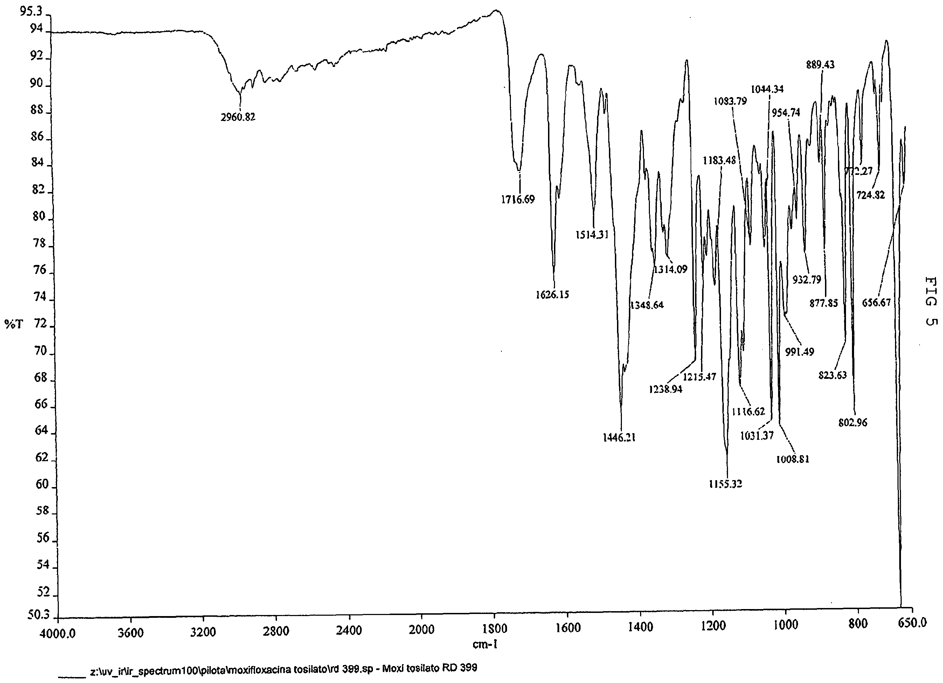

Moreover, Moxifloxacin Tosylate shows an FT-IR spectrum having the following peaks: 1717, 1626, 1446, 1239, 1155, 1031, 1009, 803 cm-1 (see Fig. 5).

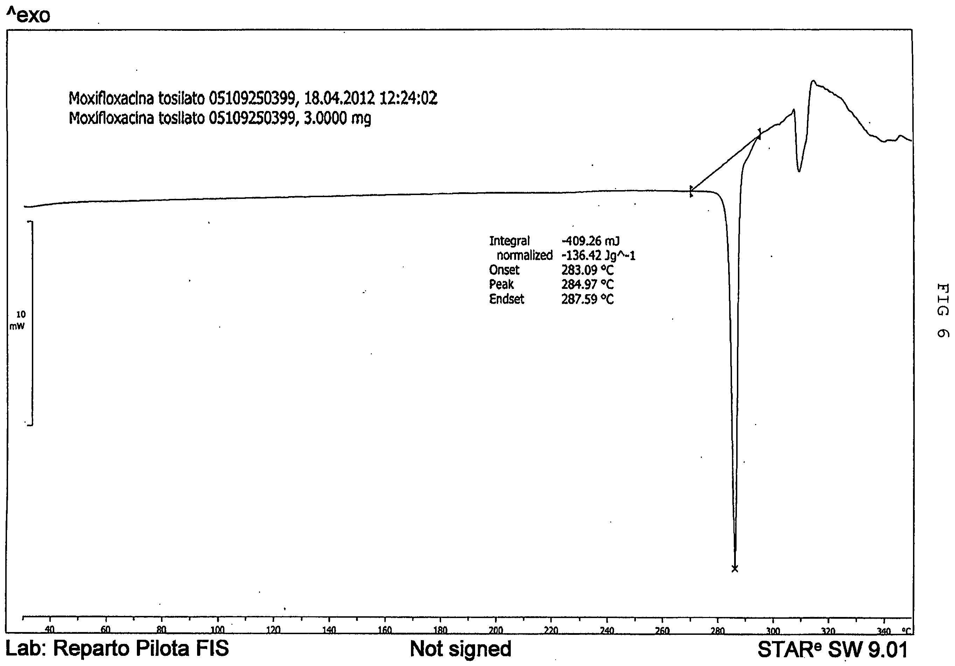

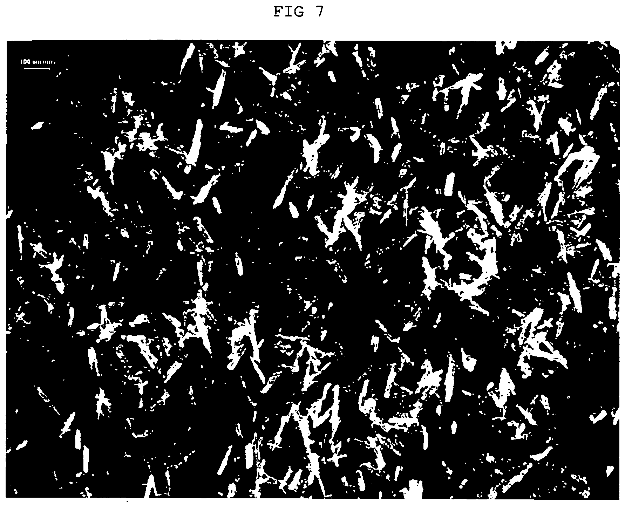

This crystalline solid has a melting point of 283°C determined by DSC (onset) (see Fig. 6) and presents a needle-shaped crystal habit (see Fig. 7).

The purified Moxifloxacin tosylate has tipically K.F. < 0,10%, Loss of Drying (LOD) < 0,20% (w/w), HPLC purity > 99.3% (A%), N-methly impurity of formula (VII) about 0.30% (HPLC A%) and specific optical rotation comprised in the range of -100° to - 106°.

Moxifloxacin hydrochloride prepared according to the method of the present invention does not contains any ppm of genotoxyc impurities such as the C1-C4 alkyl esters of p-Toluensulphonic acid, particularly methyl, ethyl and isopropyl p-Toluensulphonate. The same apply to the correspondent Moxifloxacin base and Moxifloxacin Tosylate. The GC-MS method employed for this determination is reported in example 7 and allow to detect up 0.4 ppm of these potential impurities either in Moxifloxacin salts such as the hydrochloride, Tosylate, etc. or in Moxifloxacin base.

The absence of the C1-C4 alkyl esters of p-Toluensulphonic acid or the amount lower than 10 ppm of these impurities is essential for the product Moxifloxacin hydrochloride being such impurities genotoxic and being necessary to provide a product conforms to the ICH, EMA and FDA Guidelines on Genotixic impurities. To our knowledge, none has before searched these impurities in Moxifloxacin and salts therof.

Moxifloxacin Tosylate is a process intermediate that could also be used as a product in medicine, in particular, for use as antibacterial agent and can be comprised in pharmaceutical compositions also comprising one or more pharmaceutical carries and/or excipients.

The further reduction of the amount of the N-methly impurity of formula (VII) to values below 0.15% occurs in the step of preparing Moxifloxacin hydrochloride. Or, in case of starting from Moxifloxacin containing more than 1.2% of impurity of formula (VII), it occurs in the optional purification step.

The Moxifloxacin hydrochloride obtained with the process of the present description, thus comprising the impurity of formula (VII) in a range comprised between 0.05% and 0.15% or, preferably, between 0.06% and 0.14%, or even more preferably between 0.06% and 0.09%, can be conveniently used for the preparation of pharmaceutical formulations also comprising one or more pharmaceutically acceptable excipients and/or carriers. The Moxifloxacin hydrochloride obtained according to the process of the present description can thus be used in medicine, and in particular as an antibacterial agent, given that also the formula (VII) impurity content complies with the ICH Q3A guidelines regarding the impurities in the pharmaceutical active ingredients.

The process according to the present description, according to example 3, provides Moxifloxacin hydrochloride in anhydrous form, having an HPLC purity equivalent to 99.80% and having all impurities below 0.10%, thus it has a pharmaceutically acceptable degree.

Furthermore, the compound of formula (IV) 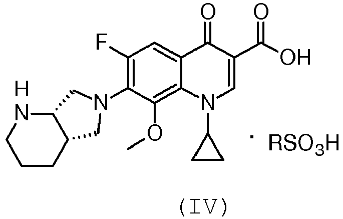 in which R is C1-C12 linear o branched alkyl or is an aryl and preferably in which R is chosen among C1-C4 alkyl, phenyl and tolyl can be used in medicine, particularly for use as antibacterial agent. Also Moxifloxacin Naphtalendisulphonate (2:1) can be used for the same medical uses, being preferred for such uses, Moxifloxacin Mesylate and most preferred, Moxifloxacin Tosylate. These compounds can be formulated in pharmaceutical compositions comprising one or more pharmaceuticals excipients and/or carriers. These formulation can be employed for oral use or for topical use being in the first case in form of tablets and in the second case in form of cream. One example of oral formulation comprises Moxifloxacin Tosyate, lactose monohydrate, microcrystalline cellulose, croscarmellose sodium, magnesium stearate, hydroxypropyl methylcellulose, titanium dioxide, polyethylene glycol.

### EXPERIMENTAL PART

### Example 1 (for description purpose) - Synthesis of Moxifloxacin Tosylate of formula (IV, R=p-MePhenyl or Tolyl) - example of the invention, step (a).

### Synthesis scheme

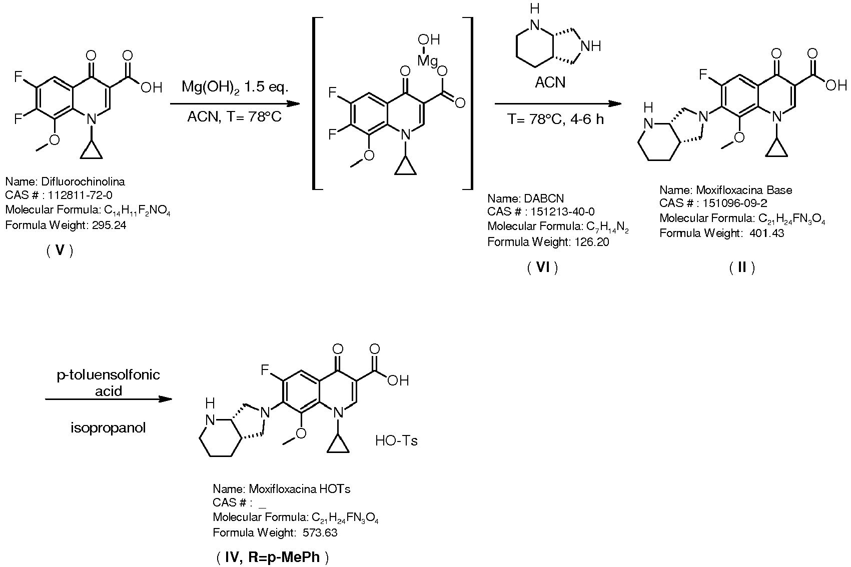

50.0 g of 1-Cyclopropyl-6,7-difluoro-1,4-dihydro-8-methoxy-4-oxo-3-quinolinic acid of formula (V) (1.0 mol. equiv.), 15.80 g of Mg(OH)₂ (1.6 mol. equiv.) and 300 mL of Acetonitrile were introduced into a 4-neck flask provided with a mechanical stirrer, thermometer, cooler and thermostat. Heating is carried out at 78°C for one hour under slow stirring with the aim of forming the Moxifloxacin complex with Magnesium. A solution formed by 34.20 g of (4aS, 7aS)-Octahydro-1H-pyrrole[3,4-b]pyridine of formula (VI) and 150 mL of Acetonitrile is dosed in 1 hour. The reaction mixture is left at reflux for another 4 hours. Upon completing the reaction (conversion exceeding 90%) the mixture is cooled at 50°C and the ACN is distilled under vacuum up to obtaining a soft pasty residue. Such residue is cooled at 0°C and a cold solution at 0°C made up of 150 mL of Acetone and 300 mL of Dichloromethane is then added slowly. Stirring is carried out for 10 minutes at room temperature then 2.5 g of dicalite and 2.5 g of acticarbone are added. Stirring is carried out for 10 minutes then filtration is carried out on a dicalite panel. The filtrate is concentrated to dryness in the rotavapor then recovered using 300 mL of dichloromethane. This organic solution is washed using a solution made up of 100 mL of a saturated NaHCO3 solution and 50 mL of purified water. The underlying organic phase containing the product is separated and the aqueous phase is re-extracted using 100 mL of dichloromethane. The organic phases are mixed again and then dried obtaining 71.0 g of Moxifloxacin base, with HPLC purity equivalent to 90.27% and with formula (VII) impurity equivalent to 1.09%.

The solid residue is recovered using 100 mL of purified water and 800 mL of Isopropanol. Heating is carried out at reflux up to complete dissolution. A solution of 26.06 g of paratoluenesulfonic acid (1.1 mol. equiv.) in 100 mL of Isopropanol is dripped at 70°C in one hour. It is slowly brought to 35°C, then it is left under stirring at 35°C for one night.

The suspension is paper-filtered at 35°C and the solid is washed using 100 mL of Isopropanol. The solid is dried in an oven at 50°C for at least 8 hours.

72.5 g equivalent to a 74.6% molar yield of Moxifloxacin Tosylate are obtained as a hazelnut-coloured solid with HPLC purity equivalent to 98.46% and with a formula (VII) impurity content equivalent to 0.54%.
¹H-NMR (400 MHz, DMSO-*d₆*) : δ/ppm = 0.84-1.24 (m, 4H), 1.70-1.85 (m, 4H), 2.30 (s, 3H), 2.65-2.2.72 (m, 1H), 2.98-3.03 (m, 1H), 3.24-3.28 (m, 1H), 3.52-3.55 (m, 1H), 3.59 (s, 3H), 3.75-3.92 (m, 3H), 4.09-4.20 (m, 2H), 7.12 (d, 1H, j=7.88 Hz), 7.49 (d, 1H, j=8.04 Hz), 7.71 (d, 1H, j=14.13 Hz), 8.69 (s, 1H).
Melting point = 273°C (determined using DSC).

### Example 2 (for description purpose) - Purification of Moxifloxacin Tosylate of formula (IV, R=p-MePhenyl or Tolyl) - example of the invention, optional step (b).

72.5 g of Moxifloxacin Tosylate obtained in Example 1 are dispersed in a solution of 1276 ml of isopropanol and 142 ml of water. The suspension is heated at 80°C for one hour, then slowly cooled for one night up to 35°C. The suspension remains at 35°C for 2 hours, then filtering is carried out a paper filter and washing with 142 ml of isopropanol. The solid is dried in an oven at 50°C for at least 8 hours.

68.2 g equivalent to a 94% molar yield of purified Moxifloxacin Tosylate as a dirty white solid are obtained. The obtained Moxifloxacin Tosylate has an HPLC purity equivalent to 99.30% (A%) with a formula (VII) impurity content equivalent to 0.27%.

### Example 3 (for description purpose) - Synthesis of Moxifloxacin Hydrochloride starting from Moxifloxacin Tosylate of formula (IV, R=p-Mephenyl)

### Synthesis scheme

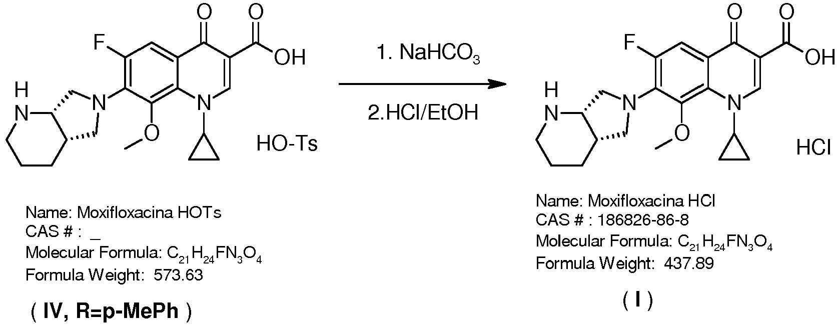

68.2 g of purified Moxifloxacin Tosylate of example 2 (1.0 mol. Equiv.) (HPLC purity equivalent to 99.30% and impurity of formula (VII) equivalent to 0.27%), 409.2 mL of saturated NaHCO3 solution, 136.4 mL of purified water, 409.2 mL of dichloromethane and 68.2 mL of isopropanol were introduced into a 4-neck flask provided with a mechanical stirrer, thermometer, cooler and thermostat. Heating is carried out at 30-35°C and stirring is carried out for one hour up to complete dissolution. Then the phases are separated and the aqueous phase is extracted using 136.4 mL of Dichloromethane. The organic phases are collected and dried. The obtained solid is recovered with 954.8 mL of Ethanol and 136.4 mL of purified water. Heating is carried out at reflux up to complete dissolution, then it is brought to 60°C and a solution of 12.3 mL of concentrated hydrochloric acid (32%) (1.05 mol. equiv.) and 136.4 mL of Ethanol is dripped in 2 hours. It is brought to r.t in 4 hours, it is left at room temperature for at least 4 hours, then it is cooled at 0°C and it is left at 0°C for another 4 hours. The obtained crystal is filtered washing it with 68.2 mL of Ethanol. The obtained salt is dried at 50°C under vacuum for 24 hours. 44.3 g of Moxifloxacin hydrochloride with a molar yield equivalent to 85.1% with HPLC purity equivalent to 99.79% and a formula (VII) impurity content equivalent to 0.08% are obtained. The obtained product does not contain, even at ppm level, any ester of the sulfonic acids as an impurity, which is an essential aspect it being widely known that such impurities are genotoxic.

### Example 4 - Preparation of the impurity of formula (VII).

### Synthesis scheme

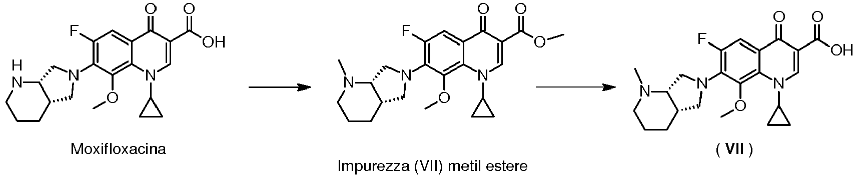

### Moxifloxacina = Moxifloxacin

### Impurezza (VII) metal estere = (VII) methyl ester impurity

5 g of Moxifloxacin free base (1.0 mol. equiv.), 50 mL of methanol and 3.5 g of potassium carbonate are introduced into a 3-neck flask provided with a mechanical stirrer, thermometer, cooler and thermostat. Cooling is carried out at 0°C and 6 g of methyl iodide (3.4 mol. equiv.) are dripped. Stirring is carried out for one night at r.t. The reaction is quenched by dripping 50 ml of a 10% sodium hydroxide solution. It is left to stir for one hour and it is brought to pH=8-9 with diluted hydrochloric acid. It is diluted using 200 ml of water and the aqueous phase is washed three times using 100 ml of dichloromethane. Precipitation is carried out by dripping 10 ml of 10% diluted hydrochloric acid, obtaining a light brown precipitate which is paper-filtered and dried in an oven at 50°C under vacuum. 4.6 g of formula (VII) impurity are obtained.
¹H-NMR (400 MHz, DMSO-*d₆*) : δ/ppm = 0.86-1.11 (m, 4H), 1.44-1.55 (m, 1H), 1.79-1.86 (m, 2H), 1.95-2.05 (m, 1H), 2.82-2.86 (m, 1H), 3.30 (s, 3H), 3.43-3.46 (m, 1H), 3.62 (s, 3H), 3.68-3.74 (m, 2H), 3.94-3.98 (m, 1H), 4.12-4.27 (m, 3H), 7.72 (d, 1H, j=13.06 Hz), 8.68 (s, 1H).

### Example 5 - HPLC method for quantifying the Moxifloxacin purity and the formula (VII) impurity content in Moxifloxacin.

### Chromatographic conditions:

| | | | |
|---|---|---|---|
| Column: | Zorbax Eclipse Plus C8, 100 x 4.6 | | |
| | mm, 1.8 µm or equivalent | | |
| Column temp: | 20°C | | |
| Mobile phase A: | TFA 0.1% | in H₂O | |
| Mobile phase B: | TFA 0.1% | in MeOH | |
| Gradient: | Time | A | B |
| | (min) | (%) | (%) |
| | 0 | 60 | 40 |
| | 5.5 | 40 | 60 |
| | 12 | 0 | 100 |
| | 13 | 0 | 100 |
| | 13.5 | 60 | 40 |
| Post-run time: | 4 minutes | | |
| Flow: | 1.1 mL/min | | |
| Detector: | UV at 306 | nm | |
| Injection volume: | 2.5 µL | | |
| Dilutant: | H₃PO₄ 0.1% in H₂O/ACN (7/3) | | |
| Duration of analysis:13.5 min | | | |

| Compound | RT (min) | RRT |
|---|---|---|
| Impurity of formula (VII) | 3.56 | 0.92 |
| MOXIFLOXACIN | 3.86 | 1.00 |

### Example 6 - Synthesis of Moxifloxacin ethanesulfonate of formula (IV, R=Et) - example of the invention, step (a).

The procedure is repeated like in Example 1 except for the use of Ethanesulfonic Acid instead of paratoluenesulfonic Acid, in the same molar equivalents. The Etanesulfonate Moxifloxacin obtained as a dirty white solid has the following 1H-NMR spectrum.
¹H-NMR (400 MHz, DMSO-*d₆*) : δ/ppm = 0.83-0.90 (m, 1H), 1.02-1.22 (m, 6H), 1.67-1.1.85 (m, 4H), 2.35-2.4 (q, 2H, j = 7.42 Hz), 2.63-2.70 (bs, 1H), 2.95-3.00 (m, 1H), 3.22-3.25 (m, 1H), 3.51-3.58 (m, 4H), 3.74-3.92 (m, 3H), 4.03-4.17 (m, 2H), 7.70 (d, 1H, j = 14.17 Hz), 8.67 (s, 1H).

### Example 7 - GC-MS method for the determination of the p-toluensulphonic acid alkyl esters (metyl, ethyl and isopropyl esters) in samples of Moxifloxacin Tosylate, Moxifloxacin hydrochloride and Moxifloxacin base.

Chromatographic conditions (GC Agilent 6890N - MS 5975 Quadrupole):
GC Column: DB 5 (or equivalent) L= 30 m ID= 0,32 mm FT= 0.25 µm, with 1m precolumn
Injector Temperature: 280°C;
Oven Program: 70°C for 1.5 min, 10°C/min to 200°C, 20°C/min to a 300°C, 300°C for 4 min;
Injector: Splitless mode, with 1.0 min of splitless time.
Carrier Gas: He, 1.8 mL/min, constant flow mode(about 8 psi at 40°C);
Detector: MS a Single Quadrupole, SIM mode;
SIM Parameter: From 6.0 to 11.20 min - 155 e 186 m/z From 11.20 to 11.75 min - 155 e 200 m/z
From 11.75 min - 155 e 214 m/z
   (Modify the acquisition ranges based on RT of the analytes)
Injection Volume: 3uL with autosampler, slow injection;
Run Time: 23.5 min;
Solvent: Toluene.

The sample of Moxifloxacine Tosylate or hydrochloride must be treated with NH₃(aq)/Toluene.

## Claims

1. Process for the preparation of Moxifloxacin hydrochloride of formula (I): 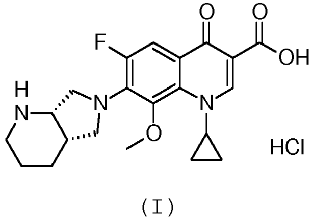 comprising the following steps:
(a) salification of Moxifloxacin base of formula (II) : 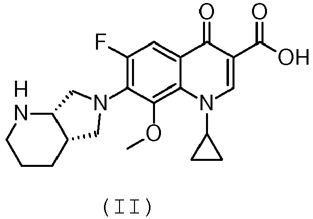 with a sulphonic acid of formula (III):
R-SO3H (III)
in which R is chosen among C1-C4 alkyl, phenyl and tolyl, to give the compound of formula (IV): 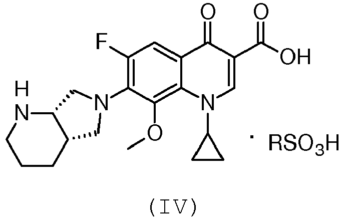
(b) optional purification of the compound of formula (IV),
(c) conversion of the compound of formula (IV) in Moxifloxacin hydrochloride of formula (I).

2. Compound of formula (IV): 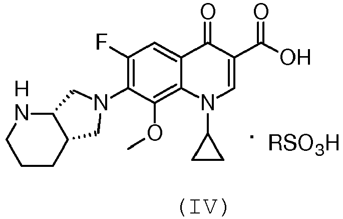 in which R is is chosen among C1-C4 alkyl and phenyl.

3. Process for the preparation of the compounds of formula (IV): 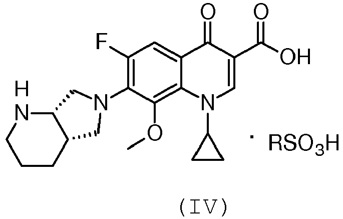 comprising the salification of Moxifloxacin base of formula (II): 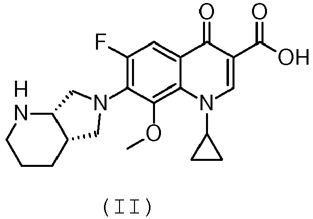 with a sulphonic acid of formula (III):
R-SO3H (III)
in which R has the same meanings given in claim 1.

4. Use of the compounds of formula (IV) according to claim 2 for the preparation of Moxifloxacin and salts thereof.

5. Use according to claim 4 in which the prepared Moxifloxacin salt is Moxifloxacin hydrochloride.

## Patentansprüche

1. Verfahren zur Herstellung von Moxifloxacinhydrochlorid mit der Formel (I): 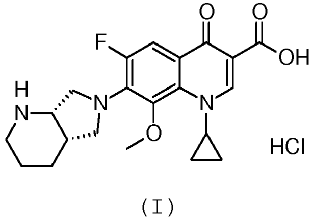 umfassend die folgenden Schritte:
(a) Salzbildung von Moxifloxacinbase mit der Formel (II) : 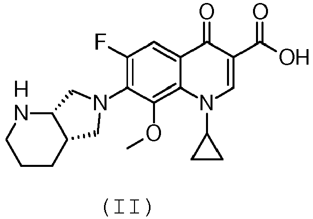 mit einer Sulfonsäure mit der Formel (III):
R-SO₃H (III)
wobei R ausgewählt ist aus C₁-C₄ Alkyl, Phenyl und Tolyl, um die Verbindung mit der Formel (IV) zu ergeben: 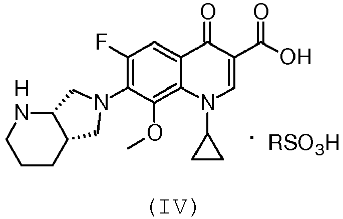
(b) gegebenenfalls Reinigung der Verbindung mit der Formel (IV),
(c) Umwandlung der Verbindung mit der Formel (IV) in Moxifloxacinhydrochlorid mit der Formel (I).

2. Verbindung mit der Formel (IV): 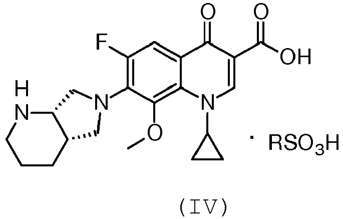 wobei R ausgewählt ist aus C₁-C₄ Alkyl und Phenyl.

3. Verfahren zur Herstellung der Verbindungen mit der Formel (IV): 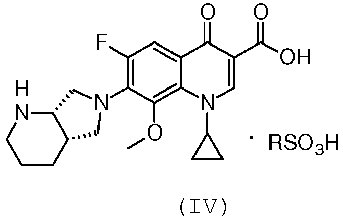 umfassend die Salzbildung von Moxifloxacinbase mit der Formel (II): 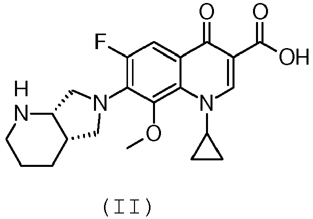 mit einer Sulfonsäure mit der Formel (III):
R-SO₃H (III)
wobei R die gleichen Bedeutungen hat, die in Beispiel 1 angegeben wurden.

4. Verwendung der Verbindungen mit der Formel (IV) gemäß Anspruch 2 zur Herstellung von Moxifloxacin und Salzen davon.

5. Verwendung nach Anspruch 4 wobei das hergestellte Moxifloxacinsalz Moxifloxacinhydrochlorid ist.

## Revendications

1. Procédé pour la préparation de chlorhydrate de moxifloxacine de formule (I): 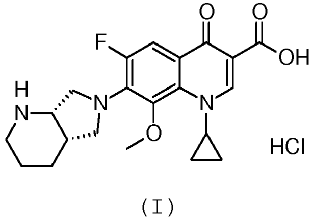 comprenant les étapes suivantes:
(a) salification de moxifloxacine base de formule (II) : 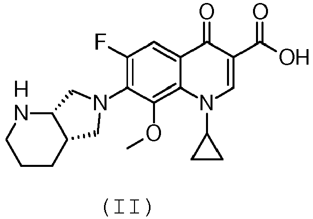 avec un acide sulfonique de formule (III):
R-SO₃H (III)
dans laquelle R est choisi parmi C₁-C₄ alkyle, phényle et toluyle, de manière à obtenir le composé de formule (IV): 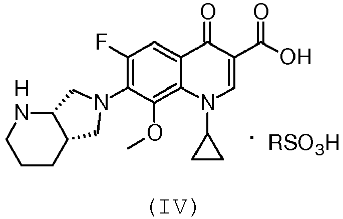
(b) purification éventuelle du composé de formule (IV),
(c) conversion du composé de formule (IV) en chlorhydrate de moxifloxacine de formule (I).

2. Composé de formule (IV): 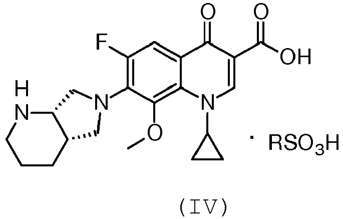 dans laquelle R est choisi parmi C₁-C₄ alkyle et phényle.

3. Procédé pour la préparation des composés de formule (IV): 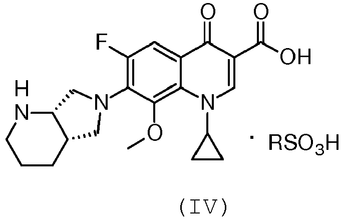 comprenant la salification de moxifloxacine base de formule (II): 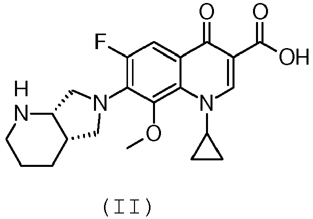 avec un acide sulfonique de formule (III):
R-SO₃H (III)
dans laquelle R a les mêmes significations que celles données dans la revendication 1.

4. Utilisation des composés de formule (IV) selon la revendication 2 pour la préparation de moxifloxacine et des sels correspondants.

5. Utilisation selon la revendication 4 dans laquelle le sel de moxifloxacine préparé est le chlorhydrate de moxifloxacine.
